# EUROPEAN PATENT SPECIFICATION

(11) **EP 3 654 984 B1**
(45) Date of publication and mention of the grant of the patent: **16.06.2021**
(21) Application number: 18756506.4
(22) Date of filing: 19.07.2018
(51) Int. Cl.: A61K 39/395, A61P 17/00, A61K 31/517, A61P 39/00, A61K 31/5377, A61K 9/00

(54) **TOPICAL TREATMENT FOR PREVENTING AND MITIGATING SKIN CYTOTOXICITY DUE TO THERAPY WITH TYROSINE KINASE INHIBITORS**
TOPISCHE BEHANDLUNG ZUR VORBEUGUNG UND LINDERUNG DER HAUTZYTOTOXIZITÄT DURCH THERAPIE MIT TYROSINKINASEINHIBITOREN
TRAITEMENT TOPIQUE POUR PRÉVENIR ET ATTÉNUER UNE CYTOTOXICITÉ CUTANÉE DUE À UNE THÉRAPIE AVEC DES INHIBITEURS DE TYROSINE KINASE

(30) Priority: 20.07.2017 IT 201700082347
(43) Date of publication of application: 27.05.2020
(73) Proprietor: EUCARE S.R.L., 00181 Roma RM (IT)
(72) Inventor: ROMANO, Maria Concetta, 00181 Roma (IT); RAVAGNAN, Giampietro, 00181 Roma (IT)
(74) Representative: Banchetti, Marina
(86) International application number: PCT/IT2018/050135
(87) International publication number: WO 2019/016843

(56) References cited:
- WO-A1-2012/129499
- US-A1- 2011 190 244
- MASCC SKIN TOXICITY STUDY GROUP ET AL: "Clinical practice guidelines for the prevention and treatment of EGFR inhibitor-associated dermatologic toxicities", SUPPORTIVE CARE IN CANCER, SPRINGER-VERLAG, DE, vol. 19, no. 8, 1 June 2011 (2011-06-01), pages 1079-1095, XP019925029, ISSN: 1433-7339, DOI: 10.1007/S00520-011-1197-6
- AMINAH JATOI ET AL: "Prophylactic tetracycline does not diminish the severity of epidermal growth factor receptor (EGFR) inhibitor-induced rash: results from the North Central Cancer Treatment Group (Supplementary N03CB)", SUPPORTIVE CARE IN CANCER, SPRINGER-VERLAG, DE, vol. 19, no. 10, 6 September 2010 (2010-09-06), pages 1601-1607, XP019947578, ISSN: 1433-7339, DOI: 10.1007/S00520-010-0988-5
- J.-B. BACHET ET AL: "Folliculitis Induced by EGFR Inhibitors, Preventive and Curative Efficacy of Tetracyclines in the Management and Incidence Rates According to the Type of EGFR Inhibitor Administered: A Systematic Literature Review", THE ONCOLOGIST, vol. 17, no. 4, 16 March 2012 (2012-03-16) , pages 555-568, XP055313906, US ISSN: 1083-7159, DOI: 10.1634/theoncologist.2011-0365
- ARRIETA OSCAR ET AL: "Randomized, open-label trial evaluating the preventive effect of tetracycline on afatinib induced-skin toxicities in non-small cell lung cancer patients", LUNG CANCER, ELSEVIER, AMSTERDAM, NL, vol. 88, no. 3, 28 March 2015 (2015-03-28) , pages 282-288, XP029590321, ISSN: 0169-5002, DOI: 10.1016/J.LUNGCAN.2015.03.019 cited in the application

## Description

### Field of the invention

The present invention relates to a topical treatment for the prevention and mitigation of skin cytotoxicity due to tyrosine-kinase inhibitory drugs, in particular, EGFR inhibitors. More specifically, the invention relates to the use of a polydatin-based topical preparation (polydatin being a glycosylated derivative of resveratrol, also known as piceid), to be applied on the skin before treatment with anti-tumor tyrosine-kinase inhibitors in order to protect the skin from the adverse skin effects of the systemic treatment with this kind of drugs.

### Background of the invention

In recent years, the new knowledge on molecular biology regarding the alterations of proliferative signals in neoplastic cells has been the rational for the development of the so-called "molecularly targeted biologic therapies", also known as "targeted therapies", which are profoundly innovative compared to traditional chemotherapy. While the chemotherapy acts, in fact, on all tumor cells through an activity of cell destruction that also affects healthy cells, the new molecularly targeted therapies interfere with some of the receptors expressed by the neoplastic cells, in particular, preventing certain tumor growth factors from entering these cells to develop them in a malignant way.

In this framework, the use of drugs that irreversibly interact with the receptors present and expressed in many solid and hematological tumors has led to considerable successes, above all due to the increase in life expectancy of the treated patients.

In the treatment protocols of many tumors many drugs that can block growth factor receptors or other protein molecules with similar activity, in particular, those of the tyrosine kinase family (TK), have been introduced. It is known that tyrosine kinases are enzymes that regulate cellular processes, such as cell proliferation and differentiation, and that they may be involved in the oncogenesis process, as they catalyze the phosphorylation of tyrosine residues of some cell receptors, including Epidermal Growth Factor Receptor (EGFR), VEGFR-1 and 2 (Vascular Endothelial Growth Factor Receptor-1 and 2), PDGFR-A, -B, -C e -D (Platelet-Derived Growth Factor), the RET proto-oncogene (tyrosine kinase receptor for ligands of the GDNF family, Glial cell-Derived Neurotrophic Factor) and the BRAF proto-oncogene (ser-ine/threonine-protein kinase B-Raf).

In particular, among the TKs that constitute the molecular target of the most established biological therapies there are firstly EGFR and the abnormal protein tyrosine-kinase Bcr-Abl. The molecules called *erlotinib, gefitinib, afatinib* and *lapatinib,* as well as the monoclonal antibodies *cetuximab* and *panitumumab,* are part of the group of drugs that irreversibly interacts with EGFR (EGF receptor tyrosine kinase inhibitors, EGFR TKI), while known drugs inhibiting the tyrosine kinase protein Bcr-Abl are *imatinib, dasatinib, nilotinib, bosu-tinib, ponatinib* and *bafetinib.*

Furthermore, a molecule capable of acting as an inhibitor of many receptors with kinase activity including VEGFR, PDGFR and Raf kinases is *sorafenib.* As a further example of a drug targeting multiple receptors, *sunitinib* is able to inhibit all VEGFR and PDGFR receptors, as well as the Raf kinases and the kinase proto-oncogene CD117.

Although the targeted agents are generally less toxic than conventional chemotherapeutic agents, the above tyrosine kinase inhibitors are associated with non-isolated troublesome side effects, primarily on the skin and intestinal mucosa, which are the direct consequence of expression of the tyrosine-kinase receptor on cells of epithelial origin. A common adverse event to treatment (particularly with imatinib, sunitinib, sorafenib, erlotinib, gefitinib and afatinib) is acneiform rash, which occurs a few days after the start of treatment and is more intense during the 2^{nd} - 3^{rd} week of therapy.

In particular, it has been hypothesized that inhibition of EGF receptors at the level of keratinocytes results in growth arrest and apoptosis, reduces cell migration and increases cell differentiation and inflammation. For example, the most common skin reactions observed during treatment with sunitinib and sorafenib are skin rash (46%), hand-foot syndrome (or palmopantar erythrodysesthesia, a disorder characterized by tingling, reddening and painful swelling of the palm of the hands and soles of the feet) (42%), folliculitis (38%), pruritus (36%), stomatitis (16%), alopecia (15%) and nail alterations (14%). According to some studies, the highest incidence of acneiform rash has been observed during erlotinib treatment.

It is evident that such side effects must be managed in most patients and can affect the regular continuation of therapy. In the most severe cases the therapy must be suspended in order not to compromise the essential functions of the person, and then it can be resumed following the regression of the lesion, normally with a reduced dose administration.

Taking into consideration, by way of example, the treatment of Non-Small Cell Lung Cancer (NSCLC), which represents 80-85% of malignant lung neoplasms, currently the optimal management of this tumor is based on the screening of some predictive and prognostic biomarkers, able to predict the sensitivity to targeted therapy and to estimate the prognosis. Targeted therapy of NSCLC relies on EGF tyrosine-kinase receptor inhibitors, EGFR-TKI in the great majority of cases.

As already noted, the epidermal growth factor receptor (EGFR, also known as ErbB1 or HER1) is a transmembrane glycoprotein of 170 kDa constitutively expressed on the surface of epithelial cells. It belongs to a family of tyrosine-kinase receptors that can initiate a wide range of signaling pathways leading to cell growth, proliferation, and survival. Three main mechanisms can activate EGFR: an increased expression of EGFR on malignant cells; an increased production of ligands by malignant cells; activating mutations of EGFR within the malignant cells. The overexpression of EGFR is strongly associated with the development and progression of several malignancies, including advanced NSCLC. In fact, EGFR is overexpressed in 40-89% of the NSCLC, and represents a promising translational therapeutic object.

However, it was subsequently discovered that the main therapeutic target is the activation of mutations rather than the overexpression of EGFR. The two most frequent mutations are deletions of exon 19 (60%) and missense substitutions L858R at position 858 (35%), where leucine is replaced by arginine, with consequent constitutive activation of the receptor without ligand binding.

The mutant EGFR, as already noted, can be inhibited by both small inhibitory molecules of TK (such as, erlotinib, gefitinib and afatinib) and monoclonal antibodies (such as, cetuximab). EGFR inhibition is now well established as an effective treatment for several tumors: first generation EGFR tyrosine kinase inhibitors, erlotinib and gefitinib, are associated with high response rates (approximately 60-70%) when given to an appropriate patient population, while afatinib is a potent second-generation irreversible ErbB family blocker, which inhibits the tyrosine kinase activity of EGFR, human epidermal growth factor 2 (HER2, ErbB2), ErbB4 and all related dimers of the ErbB family (Solca F et al., Target binding properties and cellular activity of afatinib (BIBW 2992), an irreversible ErbB family blocker. J Pharmacol Exp Ther 2012; 343: 342-350).

It is known that EGFR is expressed on the basal layer of the epidermis and plays an important role in the stimulation of epidermal growth, in the inhibition of epithelial cell differentiation and in the acceleration of wound healing. The effects of EGFR inhibition, therefore, include damage to the growth and migration of keratinocytes and the expression of inflammatory chemokines by these cells. These effects lead to inflammatory cell recruitment and resulting skin lesions, responsible for most adverse reactions, including swelling, pap-ule-pustules and periungual inflammation (Lacouture ME. Mechanisms of cutaneous toxicities to EGFR inhibitors. Nature Rev Cancer 2006; 6 (10): 803-12).

As in the case of therapy with other tyrosine kinase receptor inhibitors, typically the rash develops about 7-10 days after the start of treatment, affecting the skin above the waist, and generally resolves spontaneously without treatment. In most patients the rash is mild, but in the most severe cases (grade 3 or 4) it is recommended to discontinue the drug for 7-10 days, and recovery with a dose reduction of 25%.

In cases of severe local inflammation, a possible intervention is based on drugs able to reduce the recruitment of inflammatory cells. Starting from the consideration that systemic standard tetracycline and topical steroids are commonly used for acne treatment, the similarity between acne and the rash induced by EGFR inhibitors has suggested a possible role for tetracyclines in the treatment or prevention of skin rash resulting from treatment with such agents (Jatoi A et al. Tetracycline to prevent epidermal growth factor receptor inhibitor-induced skin rashes: results from a placebo-controlled trial from the North Central Cancer Treatment Group (N03CB), Cancer 2008; 113: 847-53).

In general, cutaneous toxicities associated with molecularly targeted biologic therapy agents are considered completely reversible, except for telan-giectasias. Their proper management is important, because they can potentially influence both the patient's quality of life and compliance with treatment, and predispose the skin to bacterial, fungal or viral infections. (Ocvirk J et al., A review of the treatment options for skin rash induced by EGFR-targeted therapies: Evidence from randomized clinical trials and a metaanalysis. Radiol Oncol 2013; 47 (2): 166-175). Therefore, it is necessary and urgent to adopt therapeutic and preventive strategies for the management of these toxicities, in order to be able to continue the treatment while maintaining maximum tolerability for the patient and to avoid delays and interruptions in the treatments.

The rashes appear mainly on the face, neck and upper trunk, and the face is often the first affected area. The rash is characterized by erythematous papules and interfollicular and follicular pustules, without the microcomedons and the comedones characteristic of acne. The rash tends to grow and shrink during therapy, with acute phases that are occasionally noticed after administration of the drug. The cutaneous symptoms tend to resolve without leaving scars after the end of treatment. The physiopathology of cutaneous rash associated with EGFR inhibitors is not yet fully understood, but interference with the proliferation, differentiation, migration and adhesion of keratinocytes during anti-EGFR therapy, resulting in recruitment of inflammatory cells and lesions to skin tissues. Because EGFR is highly expressed in epidermal keratinocytes, in the sebaceous glands and in the epithelium of the hair follicle, inhibition of these receptors may produce characteristic dermatological effects.

A consensus committee of the National Comprehensive Cancer Network (NCCN) based on expert advice has published recommendations on treatment for skin rash. Patients should use emollients for dry skin, or xerosis, which accompanies anti-EGFR therapies. A high protection factor sunscreen should be used promptly, because inhibition of EGF receptors reduces the protective nature of the receptors themselves, allowing sun exposure to worsen the symptoms of irritation. According to this document, mild rash symptoms may not require intervention and, if necessary, a clindamycin gel or topical hydrocortisone may be adequate. Symptoms of moderate rash can be managed as above, or with the combination of 1% topical pimecrolimus plus a tetracycline-related antibiotic, such as oral doxycycline 100 mg twice daily or oral minocycline 100 mg twice daily. A severe rash requires dose interruption of anti-EGFR agents, treatment with a tetracycline analogue and application of hydrocortisone cream, clindamycin gel or pimecrolimus, plus an oral dose of steroids (Burtness B el al., NCCN task force report: management of dermatologic and other toxicities associated with EGFR inhibition in patients with cancer. J Natl Compr Cancer Netw. 2009: 7 (suppl 1): S5-S21).

On the same topic, the review of the MASCC Skin Toxicity Study Group (Lacouture M.E. et al., Clinical practice guidelines for the prevention and treatment of EGFR inhibitor-associated dermatologic toxicities, Support Care Cancer 2011; 19:1079-1095) reports a set of guidelines for the prevention and treatment of dermatological toxicities associated with administration of EGFR antagonists in cancer patients. The recommendations for skin rash are similar to those already enunciated by the National Comprehensive Cancer Network and include preventive topical treatment with a cortisone cream and systemic administration of a tetracycline (minocycline or doxycycline), and treatment during therapy with a cortisone cream and a clindamycin cream (an antibiotic), as well as the systemic administration of tetracyclines and isotretinoin (an anti-acne medication).

Regarding afatinib in particular, the side effects of this drug now widely used in lung cancer are also highlighted in the official documentation of the marketing authorization of the EMA, where pathologies of skin and subcutaneous tissue are reported as "very common" (frequency ≥ 1/10) the following: skin rash, acneiform dermatitis, itching and dryness of the skin (xerosis), and as "common" (frequency ≥ 1/100 e < 1/10) the palmoplantar erythrodysesthesia syndrome.

Considering the foregoing, the present invention therefore proposes to provide a management strategy for cutaneous toxicity associated with tyrosine kinase inhibitory agents, used at a systemic level in molecularly targeted therapies, which is not only effective but also well tolerated by the patient, and which reduces as much as possible the need to resort to corticosteroids and antibiotics such as tetracyclines to cope with the dermatological adverse events occurring during these therapies.

### Summary of the invention

In the context of the studies connected with the present invention, known molecules of natural origin, the stilbenoids, and the relative oligomers and glycosides, whose best-known representative is resveratrol (3,4,5'-trihydroxystybene) have been considered.

As is known, resveratrol and the other natural stilbene compounds which are related to them are ubiquitous molecules in the plant world, and are classified as phytoalexins. The latter are a heterogeneous class of molecules whose role in plant physiology seems to be primarily the inhibition of the progression of fungal infections. These compounds are present in several plant species, among which, in particular, the *Polygonaceae* and the *Vitaceae.* From the dried roots of *Polygonum cuspidatum* a product of traditional Chinese medicine is obtained, whose activity is precisely due to the presence of resveratrol and the related compounds.

Among the many documents of scientific and patent literature on resveratrol and its derivatives, the European patent EP 1292319 B1 (CNR e Istituto Agrario di S. Michele all'Adige), of which one of the current inventors is a co-author, describes the oligomers of resveratrol as new compounds isolated from the grapevine or Polygonum extract, as well as the use of a larger group of such stilbenoids (dimers, trimers and tetramers of the cis- and trans-resveratrol, as well as the related glucose derivative referred to as piceid, resveratrol-3-O-β-mono-D-glucoside or 3,4,5'-trihydroxysylbene-3-β-mono-d-glucoside, also known as polydatin) for the treatment of colorectal carcinoma and melanoma. Moreover, the European patent EP 1292320 B1**,** of the same owners and same inventors, describes the use of a larger group of compounds of the same family for the therapy of some immunodeficiencies and autoimmune diseases, that is, with functions of immunomodulating agents.

Further studies, in particular on the polydatin/piceid glucoside, have shown that this compound has a powerful antioxidant activity, which may explain its biological properties to reduce the deleterious effects of oxidative stress on cells and on different tissues (Huang K et al., Polydatin promotes Nrf2-ARE anti-oxidative pathway through activating Sirt1 to resist AGEs-induced upregulation of fibronectin and transforming growth factor-betal in rat glomerular messangial cells. Mol Cell Endocrinol. 2015; 399: 178-189). Furthermore, the literature reports for polydatin an inhibition activity of lipid perox-idation (European patent application EP 2087894A1 by Glures S.r.l.) and a lipid synthesis modulation activity (Xing WW et al., Effects of polydatin from Polygonum cuspidatum on lipid profile in hyperlipidemic rabbits. Biomed Phar-macother. 2009; 63 (7): 457-462), as well as anti-allergic effects (Yang B et al., Polydatin attenuated food allergy via store-operated calcium channels in mast cell. World J Gastroenterol. 2013;19 (25): 3980-3989) and anti-tumoral effects in addition to those already mentioned above (Zhang Y et al. Polydatin inhibits growth of lung cancer cells by inducing apoptosis and causing cell cycle arrest. Oncol Lett. 2014; 7 (1): 295-301).

Polyphenols such as polydatin have also been reported to interfere with the EGFR system in human kerarinocytes, and this effect may be involved in the regulation of skin inflammatory processes (Pastore S et al., Plant polyphenols regulate chemokine expression and tissue repair in human keratinocytes through interaction with cytoplasmic and nuclear components of epidermal growth factor receptor system. Antioxid Redox Signal. 2012 Feb 15; 16 (4):314-28). Still more recent studies have also shown that resveratrol has a regulatory effect on the production of proinflammatory cytokines (Pastore S et al., Resveratrol induces long-lasting IL-8 expression and peculiar EGFR activation/distribution in human keratinocytes: mechanisms and implications for skin administration. PLoS ONE, 2013 Mar 8:(3).

Based on these observations, and further observations about the ability of polydatin to stimulate the production of β-defensins, particular peptides with antimicrobial action (Ravagnan G. et al., Polydatin, a natural precursor of resveratrol, induces β-defesin production and reduces inflammatory response. Inflammation 2013 Feb; 36 (1):26-34), according to the present invention it is proposed to use topical polydatin as prophylaxis, i.e. upstream of the systemic administration of anti-tumor agents, inhibitors of tyrosine kinase receptors, and in particular upstream of the administration of anti-EGFR drugs.

It has been found that polydatin, appropriately applied on the skin of patients who must undergo therapy with TK inhibitor molecularly targeted drugs, and in particular with anti-EGFR agents, effectively protects from the cytotoxic action that such drugs normally exert at the skin level, allowing to mitigate the skin rash and other adverse dermatological events that have been described previously. It should be noted that the clinical data obtained from the experimentation of the present invention show not so much that polydatin is able to exert an anti-inflammatory action on skin rash, rather that the preventive application of polydatin on the skin results in a reduction of the establishment of the process of cutaneous injury, and often in the non-manifestation of cytotoxicity, in a highly significant percentage as compared to the data reported in the literature.

Without wishing to be bound to any particular theory on the mechanism of action of the proposed prophylactic treatment with polydatin, it is hypothesized that the molecule of this agent, hereinafter represented in the trans-isomeric form, 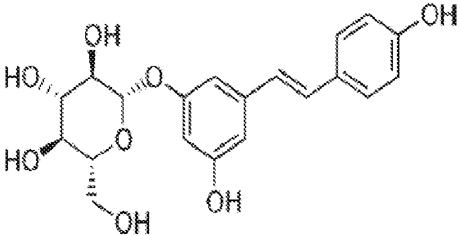 interacts with the tyrosine-kinase receptors of the cutaneous tissues, and in particular with EGF receptors, occupying them in such a way as to make them unavailable for binding with the antitumor inhibitor. In this way polydatin would protect the normal and physiological turnover of the cells of the epidermis, subtracting them from the effects that the TK inhibitory drug of would have on them (in that they are rapidly proliferating cells). In practice polydatin, interacting with the receptor even if not in a stable manner, would hinder the binding of the antitumor drug with the receptor: therefore, for example in the case of EGFR, the skin tissue, without EGFR blocking, could perform its normal functionality of continuous generation of new cells by the keratinocytes, maintaining its normal trophic level.

### Detailed description of the invention

Therefore the present invention specifically concerns a cutaneous topical preparation based on polydatin (3,4,5'-trihydroxystilbene-3-β-mono-d-glucoside) in a suitable vehicle for skin application or for application on oral mucosa for the use in the prevention and/or mitigation of cutaneous or mucosal reactions adverse to therapy with tyrosine kinase inhibitory antitumor drugs, in which the preparation is applied to the skin or oral mucosa, one or more times a day before and/or during therapy with said drugs, preferably starting the application at least 24 hours before the start of therapy with tyrosine kinase inhibitors antitumor drugs, before and/or during therapy with said drugs.

It should be noted that the topical use of polydatin does not interfere with the molecule of the antitumor drug taken systemically, which can play its oncocytotoxic role in the affected organs without causing the collateral phenomena of dermatological cytotoxicity.

According to specific embodiments of the invention, the adverse skin reactions for which it is proposed to use the topical treatment of prophylaxis based on polydatin belong to the group consisting of: skin rash, acneiform dermatitis, hitching, nail changes, skin xerosis, palmo-plantar erythrodysesthesia, folliculitis, stomatitis and alopecia.

Preferably, the antitumor tyrosine kinases inhibitor drugs whose adverse reactions are treated, according to the invention, by the application of a topical preparation based on polydatin are the inhibitors of EGF tyrosine-kinase receptor (EGFR TKI), also known as EGFR inhibitors or anti-EGFR drugs, and in particular both the small inhibitory molecules of these receptors and the monoclonal antibodies directed against the same receptors. Specifically, according to some preferred embodiments of the invention, said tyrosine-kinase inhibitors of EGF receptors, whose cytotoxic effects are addressed and, above all, prevented, with the application of topical polydatin, are selected from the group consisting of erlotinib, gefitinib, afatinib and lapatinib, and in the monoclonal antibodies cetuximab and panitumumab.

According to a specific embodiment of the invention, which will be illustrated in detail in the experimental part that follows, the tyrosine kinase inhibi-tor of EGF receptors, against whose adverse effects it is proposed the use of the prophylactic treatment with polydatin topical, is afatinib, second generation anti-EGFR drug indicated, possibly in association with other antineoplastic drugs, for the treatment of some forms of lung carcinoma.

The preparation based on polydatin to be used according to the invention can be in any of the suitable forms, respectively, for the topical application on the skin or on the mucous membranes of the oral cavity which have already been proposed for other uses, for example for use as an oxidant or as scavenger agent of ROS (reactive oxygen species) in cosmetic dermatology. Preferably, such a topical preparation comprises from 0.1 to 5% by weight of polydatin, and in a particularly preferred manner has a polydatin content of between 1 and 2% by weight.

The preparation containing polydatin may be carried in any of the pharmaceutical forms normally used or usable for topical application on the skin or oral mucosa. In particular, it may be in the form of aqueous solution or suspension, an oil-in-water or water-in-oil or multiple emulsion, a microemulsion or a nanoemulsion, or aqueous gel, depending on the excipients used for the formulation. The application on the skin or mucous membranes can be in the form of milk, spray, lotion, serum, cream, ointment or gel, containing polydatin appropriately diluted in a vehicle suitable for skin application (or on the mucosa of the oral cavity, in the case of application for the prophylaxis of adverse events affecting oral drugs, such as stomatitis/mucositis), together with any co-formulants and other excipients selected from those known and used in pharmaceutical and cosmetic techniques.

Some exemplary formulations for the preparation proposed for the use according to the invention are reported hereinafter.

According to a preferred embodiment of the invention, the proposed preparation to be used for prophylaxis of adverse skin reactions to tyrosine kinase inhibitory antitumor drugs is in the form of a cream containing from 1 to 2% by weight of polydatin, preferably 1.5% by weight of polydatin.

Preferably, according to the experimental protocol illustrated below, in the prophylactic use considered here, the topical preparation based on polydatin is applied one, or better, twice a day on the face and on the whole body, including the periungual area, at least at starting from the first day of treatment with said tyrosine kinase inhibitor antitumor drug. To best exploit the prophylactic action of the product, the application preferably begins at least a week before the treatment with the antitumor drug.

### Formulation examples

In the following examples the ingredients are identified with the INCI nomenclature. In the following examples, all percentage units are by weight.

### Example 1

### Formula A: Liposomes in water, polydatin for dermatology

Lecithin (5-10%), Oleyl erucate (5%), Polydatin (0.5-2%), Tocopherol acetate (3%), Magnesium sulphate (2%), Ethylexylglycerin (0.5%), Phenoxyethanol (0.6%).

### Example 2

### Formula B: Liposomes in water, polydatin for dermatology

Lecithin (5-10%), Dicaprylyl ether (5%), Polydatin (0.5-2%), Tocopherol acetate (3%), Magnesium sulphate (2%), Ethylexylglycerin (0.5%), Phenoxyethanol (0.6%).

### Example 3

### Formula C: emulsion in water, polydatin for dermatology

Emulsifier: Tego care PBS 6 (2-4%), Caprylic/capric triglycerides (10%), Stearyl and cetyl alcohols (2%), Cetyl palmitate (2%), Polydatin (0.5-2%), Human-like Ceramides (SK-INFLUX) (2%), Tocopherol acetate (2%), Thickeners (0.4%), pH correctors (0.1%), Preservative mix (0.6%), Phenoxyethanol (0.6%).

### Example 4

### Formula D: emulsion in water, polydatin for dermatology

Emulsifier: Tego care PBS 6 (2-4%), Caprylic/capric triglycerides (10%), Stearyl and cetyl alcohols (2%), Cetyl palmitate (2%), Octyldodecanol (3%), Polydatin (0.5-2%), Human-like Ceramides (SK-INFLUX) (2%), Tocopherol acetate (2%), Dicaprylyl ether (0,3%), Oleyl erucate (0.3%), Thickeners (0.4%), pH correctors (0.1%), Preservative mix (0.6%), Phenoxyethanol (0,6%).

### Example 5

### Anhydrous formula E: with polydatin for dermatology

Cegesoft VPB (BASF) (74.5-84.5%), Shea butter (20%-10%), Dicaprylyl ether (5%), Polydatin (0.5%-2%), Tocopherol acetate (3%).

### Example 6

### Anhydrous formula F: with polydatin for dermatology

Shea butter (94.5-92.5%), Dicaprylyl ether (2%), Polydatin (0.5-2%), Tocopherol acetate (3%).

### Example 7

### Formula G: emulsion in water, polydatin for dermatology

Emulsifier: Tego care PBS 6 (2-4%), Caprylic/capric triglycerides (10%),

Stearyl and cetyl alcohols (2%), Cetyl palmitate (2%), Octyldodecanol (3%), Polydatin (0.5-2%), Hyaluronate sodium 2% (1%-3%), Human-like Ceramides (SK-INFLUX) (2%), Tocopherol acetate (2%), Dicaprylyl ether (0.3%), Oleyl erucate (0.3%), Thickeners (0.4%), pH correctors (0.1%), Preservative mix (0.6%), Phenoxyethanol (0.6%).

### Example 8

### Formula H: emulsion in water, polydatin for dermatology

Emulsifier Tego care PBS 6 (2-4%), Caprylic/capric triglycerides (10%), Stearyl and cetyl alcohols (2%), Cetyl palmitate (2%), octyldodecanol (3%), Polydatin (0.5-2%), N-Acetylcysteine (0.1-0.5%), Human-like Ceramides (SK-INFLUX) (2%), Tocopherol acetate (2%), Dicaprylyl ether (0.3%), Oleyl erucate (0.3%), Thickeners (0.4%), pH correctors (0.1%), Preservative mix (0.6%), Phenoxyethanol (0.6%)

Some experimental results obtained with the application of a specific embodiment of the present invention, including the clinical data related to the therapeutic results obtained with the application of the invention, are referred to merely as an example in the following.

### Experimental study on the impact of prophylactic dermatological treatment with polydatin on cutaneous toxicity of afatinib in patients with metastatic lung cancer

### Patients and methods

### Patients selection

Adult patients (age ≥ 18 years) with documented histologic or cytological diagnosis of stage IV metastatic non-small cell lung cancer (NSCLC) harboring common EGFR mutations and an ECOG (Eastern Cooperative Oncology Group) performance status from 0 a 2, able to receive a first-line treatment with afatinib 40 mg/day for at least 3 months, were admitted to the study.

The main exclusion criteria were: poor patient compliance; allergy or sensitivity to polydatin; current or previous treatment with topical or oral antioxidant drugs; concomitant skin diseases.

### Design of the study and treatments

The retrospective study was specifically designed to evaluate a proactive management of EGFR-mediated skin toxicity, in order to minimize the possibility of having to reduce the dose or suspend the antitumor treatment.

Patients with NSCLC with activating EGFR mutations that were required to receive a first-line treatment with afatinib were subjected to primary dermatological examination and received daily protective skin treatment as early as 24 hours before their first dose of afatinib. This treatment included a sunscreen SPF 30 UVA/UVB, a cleansing of the skin with vegetable oils emulsified in water, in order to guarantee a "similar" cleansing that avoided the depletion of the hydrolipidic film, and a cream based on 1.5% of polydatin to be applied twice a day on the face and on the whole body (including the periungual area).

Patients were monitored every 7 days for the first month, and thereafter every 20 days or when needed.

The study was approved by the ethics committee at the participant center and was conducted in accordance with the Helsinki Declaration (version 2000) and with the Good Clinical Practice guidelines.

Data about severity, duration and management of cutaneous toxicity induced by TKI were analyzed in all patients treated using a descriptive statistic.

### Endpoint

The primary endpoint was to evaluate whether proactive dermatological management (appropriate skin care plus prophylactic therapy with a polydatin-based cream) of patients with NSCLC treated with afatinib was able to reduce cutaneous toxicity rates compared to those reported in the literature. The scale used for the classification of cutaneous toxicities induced by EGFR-targeted therapies was the classification scale of the Common Terminology Criteria for Adverse Events of the National Cancer Institute. (NCI-CTCAE), version 3.0, shown in Table 1 below.

**TABLE 1**

| Classification of cutaneous toxicity according to the Common Terminology Criteria for Adverse Events of the National Cancer Institute (NCI-CTCAE) | |
|---|---|
| Grade | Description |
| 1 | Papules and/or pustules covering <10% of the body surface, which may or may not be associated with symptoms of itching or sensitivity |
| 2 | Papules and/or pustules covering 10-30% of the body surface area, which may or may not be associated with symptoms of itching or sensitivity; associated with psychosocial impact; limiting the instrumental daily activities |
| 3 | Papules and/or pustules covering > 30% of the body surface, which may or may not be associated with symptoms of itching or sensitivity; limiting daily personal care activities; associated with local supra-infections with indication of oral antibiotics |
| 4 | Papules and/or pustules that completely cover the body surface, which may or may not be associated with symptoms of itching or sensitivity and are associated with over extensive infections with indication of intravenous antibiotics; potentially deadly consequences |
| 5 | Death |

### Results

36 patients treated between January 2015 and December 2015 in the Oncology Pneumology Unit of the San Camillo-Forlanini Hospital, Rome, were enrolled in this study, but only 34 patients were suitable for the analysis. Table 2 below shows the main characteristics of the patients. In particular, 75% of patients were over 65 years of age, and 91% were non-smokers.

**TABLE 2**

| Patients characteristics (n = 34) | | | |
|---|---|---|---|
| | | Number of patients | Percent (%) |
| AGE | < 65 | 6 | 25 |
| | > 65 | 28 | 75 |
| GENDER | Female | 18 | 53 |
| | Male | 16 | 47 |
| MUTATION | exon 19 | 22 | 65 |
| | exon 21 | 12 | 35 |
| ECOG | 0-1 | 30 | 88 |
| | 2 | 4 | 12 |
| SMOKING STATUS | Smokers | 3 | 9 |
| | Non Smokers | 31 | 91 |

The average follow-up period was 6 months. The duration of treatment was calculated from the time of initiation of treatment to the date on which the treatment was discontinued, and the average duration was 6.4 months.

The study showed that the prophylactic cutaneous treatment with a cream based on 1,5% of polydatin. The incidence of rash (all grades) was 41.2%; in particular, the incidence of grade 2 rash was 20.6%, and no case of grade 3 rash was observed. It should be noted that no patient had to discontinue therapy due to toxicity of the antitumor drug.

The overall results of the study are summarized in Table 3 below.

**TABLE 3**

| Incidence of skin rash | | | | |
|---|---|---|---|---|
| | *All grades (n,* %*)* | *Grade 1 (n,* %*)* | *Grade 2 (n,* %*)* | *Grade 3 (n,* %*)* |
| *Cutaneous rash* | 14 (41.2) | 7 (20.6) | 7 (20.6) | 0 |
| *Females* | 8 (23.5) | 2 (5.9) | 3 (8.6) | 0 |
| *Males* | 6 (17.6) | 5 (14.7) | 4 (12) | 0 |

### Discussion

This study evaluated the activity of polydatin, a well tolerated natural extract, for the prophylaxis of skin toxicity during an anti-tumor treatment based on afatinib, avoiding the use of antibiotics.

Polydatin has a strong antioxidant and free radical scavenger effect, and it is known that it can neutralize cellular damage responsible for the development of chronic diseases and aging. It is also known that polydatin is more effective than resveratrol both *in vitro* and *in vivo.*

In the present study, the incidence of rash (all grades) was 41.2% (20.6% of grade 2), with no grade 3 cases and no suspension of treatment for adverse skin events. It should be noted that these figures are lower than those reported in the literature.

In fact, in clinical studies on afatinib for treatment of patients affected by cancer, the overall incidence of rash was variable between 60% and 80%. Differences in test protocols and patient populations may make it difficult to compare the incidence of the rash between one agent and another, or between trials on the same agent. Two of the largest studies in this patient population, named LUX-Lung 3 and LUX-Lung-6, respectively, have a similar design, with the exception of the comparison protocol with conventional platinum-based chemotherapy: pemetrexed/cisplatin in LUX-Lung 3 and gemcitabine/cisplatin in LUX-Lung 6 (Sequist LV et al., Phase III study of afatinib or cisplatin plus pemetrexed in patients with metastatic lung adenocarcinoma with EGFR mutations. J Clin Oncol. 2013; 31 (27): 3327-3334; Wu YL et al., Afatinib versus cisplatin plus gemcitabine for first-line treatment of Asian patients with advanced non-small-cell lung cancer harboring EGFR mutations (LUX-Lung 6): an open-label, randomized phase 3 trial. Lancet Oncol. 2014; 15 (2): 213-222).

Both studies cited reached the primary goal of progression-free survival for the group of patients whose tumors have common EGFR mutations and which received afatinib as first-line therapy. The most common (grade 3 and 4) adverse events associated with treatment with afatinib compared to chemotherapy were rash/acne, diarrhea, paronychia and stomatitis/mucositis, as shown in Table 4 below.

In the LUX LUNG 3 study, treatment was discontinued due to treatment-related adverse events in 8% of patients. The incidence of rash (all grades) was 89.1%, in particular 72.9% rash grade 1-2 and 16.2% rash grade 3.

In the LUX LUNG 6 study, the rate of treatment discontinuation due to adverse events was lower than that of LUX LUNG 3 (2.1%), as was the incidence of skin rash: all grades 80.8%, and, in particular, 66.1% rash grade 1-2 and 14.2%, rash grade 3. This could be explained by the fact that the treating physicians have become quite adept at managing the side effects of afatinib, and that what was previously perceived as higher grade toxicity was then considered to be of lesser severity.

**TABLE 4**

| More frequent adverse events due to treatment - Studyes LUX-Lung 3 and LUX-Lung 6 (Sequist LV et al., e Wu YL et al., already cited) | | | | | | |
|---|---|---|---|---|---|---|
| | LUX-Lung 3/LUX-Lung 6, % | | | | | |
| | Afatinib LL3 (n = 229) / LL6 (n = 239) | | | Pem-Cis LL3 (n = 111) / LL6 (n = 113) | | |
| | All grades | Grade 3 | Grade 4 | All grades | Grade 3 | Grade 4 |
| Rash/acne | 89.1/80.8 | 16.2/14.2 | 0.0/0.4 | 6.3/8.8 | 0 | 0 |
| Diarrhea | 95.2/88.3 | 14.4/5.4 | 0 | 14.3/10.6 | 0 | 0 |
| Paronychia/periungual problems | 56.8/32.6 | 11.4/0.0 | 0 | 0/0 | 0 | 0 |
| Stomatitis/mucositis | 72.1/51.9 | 8.37/5.4 | 0.4/0.0 | 15.3/5.3 | 0.0/0.0 | 0 |
| Reduction of appetite | 20.5/10.0 | 3.1/1.3 | 0 | 53.2/40.7 | 2.7/1.8 | 0 |
| Vomiting | 17.0/9.6 | 3.1/0.8 | 0 | 42.3/80.5 | 2.7/15.9 | 0.0/3.5 |
| Asthenia | 17.5/10.0 | 1.3/0.4 | 0 | 46.8/36.3 | 12.6/0.9 | 0 |
| Nausea | 17.9/7.5 | 0.9/0.0 | 0 | 65.8/75.2 | 3.6/7.1 | 0.0/0.9 |
| Dry skin | 29.3/NA | 0.4/NA | 0 | 1.8/NA | 0 | 0 |
| Hitching | 18.8/10.9 | 0.4/0.4 | 0 | 0.9/0.0 | 0 | 0 |

| | | | | | | |
|---|---|---|---|---|---|---|
| Pem = pemetrexed; Cis = cisplatin; NA = not available | | | | | | |

A "real life" study in 2015 evaluated the incidence and management of skin rash due to afatinib. The open-label, randomized and controlled study evaluated the prophylactic effect of tetracycline compared to no treatment in reducing rash in 90 patients with non-small cell lung cancer (NSCLC) who received afatinib 40 mg/day for 90 days. The incidence of rash of any grade ≥ 2 was lower in the tetracycline-treated arm than in the control arm: 44.5% vs 75.6% (p = 0.046) and 15.6% vs 35.6%, (p = 0.030) (Arrieta O. et al., Randomized, open-label trial evaluating the preventive effect of tetracycline on afatinib induced-skin toxicities in non-small cell lung cancer patients. Lung Cancer. 2015 Jun; 88 (3): 282-8). It should be noted that this study is subsequent, and superior in terms of the number of patients treated, compared to that in 2010 reported an absence of effect for the use of polylactic tetracycline in the treatment of cutaneous rash associated with EGFR administration (Aminah Jatoi et al., "Polylactic tetracycline does not diminish the severity of epidermal growth factor receptor (EGFR) inhibitor-induced rash: results from the North Central Cancer Treatment Group (Supplementary N03CB)", Support Care Cancer, 2010, 19:1601-1607).

Considering the most recent study, it can be seen that the pro-active effect of polydatin as reported in Table 3 (41.2% of total incidence of skin rash, of which 20.6% of grade 1, 20.6 % of grade 2 and no case of grade 3) is equivalent to that of prophylaxis with oral tetracyclines and more recent analogs thereof which, as is known (and despite some conflicting experimental data, such as the one cited above), represents a recognized therapeutic strategy for the prevention and treatment of anti-EGFR drugs.

Recently, the international multi-center open-label, exploratory, randomized and controlled study phase IIB LUX LUNG 7 (Park K et al., Afatinib versus gefitinib as first-line treatment of patients with EGFR mutation-positive non-small-cell lung cancer (LUX-Lung 7): a phase 2B, open-label, randomized controlled trial. Lancet Oncol. 2016 May; 17 (5): 577-89) evaluated patients not previously treated, affected by stage IIIB or IV NSCLC and having a common EGFR mutation (exon 19 deletion o Leu858Arg) who received treatment with afatinib 40 mg/day or with gefitinib 250 mg/day.

**TABLE 5**

| Most frequent adverse events due to treatment - Study LUX-Lung 7 (Park et al., already cited) | | | | |
|---|---|---|---|---|
| | **Afatinib (n = 160)** | | **Gefitinib (n = 159)** | |
| **Category of adverse event, %** | All | Grade 3 | All | Grade 3 |
| Diarrhea | 90.0 | 11.9† | 61.0 | 1.3 |
| Rash/acne* | 88.8 | 9.4 | 81.1 | 3.1 |
| Stomatitis* | 64.4 | 4.4 | 23.9 | - |
| Paronychia* | 55.6 | 1.9 | 17.0 | 0.6 |
| Xerosis | 32.5 | - | 37.1 | - |
| Itching | 23.1 | - | 22.6 | - |
| Asthenia* | 20.6 | 5.6 | 14.5 | - |
| Reduction of appetite | 16.3 | 0.6 | 11.9 | - |
| Nausea | 16.3 | 1.3 | 13.8 | - |
| Alopecia | 10.6 | - | 15.1 | - |
| Vomiting | 10.6 | - | 3.8 | 0.6 |
| ALT increased | 9.4 | - | 23.9 | 7.5‡ |
| AST increased | 6.3 | - | 20.8 | 2.5 |

| | | | | |
|---|---|---|---|---|
| † plus 1 case of grade 4 diarrhea ‡ plus 1 case of increased ALT grade 4 † plus 1 case of grade 4 diarrhea ‡ plus 1 case of increased ALT grade 4 ALT, alanine aminotransferase; AST, aspartate aminotransferase ALT, alanine aminotransferase; AST, aspartate aminotransferase * Term used for a group of adverse events | | | | |

As it can be seen from Table 5, the most frequent grade 3 or 4 adverse events were diarrhea (20 of the 160 patients treated with afatinib, i.e. 13%, and 2 of the 159 patients treated with gefitinib, i.e. 1%) and rash or acne (15 of the 160 patients treated with afatinib, i.e. 9%, and 5 of the 159 patients treated with gefitinib, i.e. 3%)

Finally, it should be noted that the strategy of minimization (dose reduction or temporary suspension of treatment for the management of EGFR-mediated skin toxicity could compromise the clinical benefit of treatment with EGFR TKI.) In the study conducted with the treatment of prophilaxis with polydatin according to the invention, on the other hand, only 35.3% of patients (n = 12) had to reduce the dose of afatinib from 40 to 30 mg/day due to intestinal toxicity, while no patient had to reduce the dose for the cutaneous toxicity of the drug.

Although on a limited sample of patients, the results of the experimentation according to the invention shown in Table 3 indicate that proactive management consisting of prophylactic cutaneous treatment with polydatin-based cream may reduce the incidence of cutaneous toxicity of grade 2-3 or higher without further side effects in patients treated with afatinib compared to the toxicity data of the LUX LUNG 3 and 6 studies. It is evident which is the advantage of a topical treatment such as that proposed according to the present invention as an alternative to the systemic administration of antibiotic drugs such as tetracyclines and analogs thereof (doxycycline, minocycline, etc.).

It is important to note that, according to the present invention and since that polydatin stimulates the production of β-defensin which themselves exert an antibacterial activity (Ravagnan G et al., 2013, cited above), it was not necessary to use antibiotics in treated patients, with an obvious clinical advantage. Furthermore, the topical use of polydatin does not contrast with the action at the systemic level of the drug, because the absorption by the dermal route is negligible. Polydatin certainly interacts with the EGFR receptor, impeding the stability of EGFR binding with afatinib at cutaneous level and leaving unchanged the normal replacement of the epidermal tissue, and this property of polydatin allows the regular therapeutic pathway, and therefore the elimination of dermatological complications, and gives greater possibility of obtaining the expected result in cancer therapy with anti-EGFR drugs.

It is assumed that polydatin has this property with all drugs that interact with EGFR, especially if treatment is also preventive, that is, it starts 24 hours to a week before the first administration of the tyrosine kinase receptor inhibitor anticancer drug, because it avoids the establishment of inflammatory processes. This obviously does not conflict with the effectiveness of polydatin if an inflammatory process occurs with the pharmacological treatment.

The present invention has been described with particular reference to some embodiments thereof but it should be understood that changes and modifications can be made by those skilled in the art without departing from the scope of the invention as described in the appended claims.

## Claims

1. A topical preparation comprising resveratrol-3-β-mono-D-glucoside (polydatin or piceid, or 3,5,4'-trihydroxystilbene-3-O-β-D-glucopiranoside), in a carrier suitable for application on the skin or on oral mucous membranes, for use in the prevention and/or mitigation of cutaneous or mucosal adverse reactions in the therapy with tyrosine kinase inhibitors as anticancer drugs, wherein said preparation is applied on the skin or on the oral mucous membranes once or more times a day, before and/or during the therapy with said drugs.

2. A topical preparation for use according to claim 1, wherein said preparation is applied on the skin or on oral mucous membranes once or more times a day starting at least 24 hours before the first treatment with said tyrosine kinase inhibitor anticancer drug.

3. A topical preparation for use according to claims 1 or 2, wherein said adverse reactions are selected from the group consisting of: skin rash, acneiform dermatitis, itching, ungual alterations, cutaneous xerosis, palmoplantar erythrodysesthesia, folliculitis, stomatitis, alopecia.

4. A topical preparation for use according any one of claims 1-3, wherein said tyrosine kinase inhibitor anticancer drugs are tyrosine kinase inhibitors of the EGF receptors (EGFR-TKI).

5. A topical preparation for use according to claim 4, wherein said tyrosine kinase inhibitors of the EGF receptors are selected from the group consisting of: erlotinib, gefitinib, afatinib and lapatinib, and of the monoclonal antibodies cetuximab and panitumumab.

6. A topical preparation for the use according to claim 5, wherein said tyrosine kinase inhibitor of the EGF receptors is afatinib.

7. A topical preparation for use according to any one of the preceding claims, wherein said topical preparation comprises from 0.1 to 5% by weight of polydatin.

8. A topical preparation for use according claim 7, wherein said topical preparation comprises from 1 to 2% by weight of polydatin.

9. A topical preparation for use according to any one of the preceding claims, wherein said preparation is in the form of a solution, a milk, a spray, a lotion, a serum, a cream, an ointment or a gel, containing polydatin as active ingredient.

10. A topical preparation for use according to claim 9, wherein said preparation is in the form of a cream containing from 1% to 2% by weight of polydatin, preferably 1,5% of polydatin.

11. A topical preparation for use according to any one of the preceding claims, wherein said preparation is applied at least once day on the face and the whole body, including the periungual area.

12. A topical preparation for use according claim 11, wherein said preparation is applied starting from at least 24 hours before the first day of treatment with said tyrosine kinase inhibitor anticancer drug.

13. A topical preparation for use according to claim 12, wherein said preparation is applied at least once a day on the face and the whole body, including the periungual area, starting from one week before the first day of treatment with said tyrosine kinase inhibitor anticancer drug.

14. A topical preparation for use according to any one of the claims 11-13 wherein said preparation is applied twice a day.

## Patentansprüche

1. Topische Zubereitung umfassend Resveratrol-3-β-mono-D-glucosid (Polydatin oder Piceid oder 3,5,4'-Trihydroxystilben-3-O-β-D-glucanopiranosid), in einem Träger, der zur Anwendung an der Haut oder an Mundschleimhautmembranen geeignet ist, zur Verwendung in der Vorbeugung und/oder Linderung kutaner oder mukosaler Nebenwirkungen in der Therapie mit Tyrosinkinaseinhibitoren als Antikrebsarzneimittel, wobei die Zubereitung auf die Haut oder auf die Mundschleimhautmembrane einmal oder mehrmals täglich vor und/oder während der Therapie mit den Arzneimitteln aufgetragen wird.

2. Topische Zubereitung zur Verwendung nach Anspruch 1, wobei die Zubereitung auf die Haut oder auf Mundschleimhautmembrane einmal oder mehrmals täglich beginnend mindestens 24 Stunden vor der ersten Behandlung mit dem Tyrosinkinaseinhibitor-Antikrebsarzneimittel aufgetragen wird.

3. Topische Zubereitung zur Verwendung nach einem der Ansprüche 1 oder 2, wobei die Nebenwirkungen ausgewählt sind aus der Gruppe bestehend aus: Hautausschlag, akneähnlicher Dermatitis, Jucken, Veränderungen der Nägel, kutaner Xerose, palmoplantarer Erythrodysesthesie, Folliculits, Stomatitis, Alopezie.

4. Topische Zubereitung zur Verwendung nach einem der Ansprüche 1-3, wobei die Tyrosinkinaseinhibitor-Antikrebsarzneimittel Tyrosinkinaseinhibitoren der EGF-Rezeptoren (EGFR-TKI) sind.

5. Topische Zubereitung zur Verwendung nach Anspruch 4, wobei die Tyrosinkinaseinhibitoren der EGF-Rezeptoren ausgewählt sind aus der Gruppe bestehend aus: Erlotinib, Gefitinib, Afatinib und Lapatinib und den monoklonalen Antikörpern Cetoximab und Panitumumab.

6. Topische Zubereitung zur Verwendung nach Anspruch 5, wobei der Tyrosinkinaseinhibitor der EGF-Rezeptoren Afatinib ist.

7. Topische Zubereitung zur Verwendung nach einem der vorstehenden Ansprüche, wobei die topische Zubereitung 0,1 bis 5 Gewichtsprozent Polydatin umfasst.

8. Topische Zubereitung zur Verwendung nach Anspruch 7, wobei die topische Zubereitung 1 bis 2 Gewichtsprozent Polydatin umfasst.

9. Topische Zubereitung zur Verwendung nach einem der vorstehenden Ansprüche, wobei die Zubereitung in der Form einer Lösung, einer Milch, eines Sprays, einer Lotion, eines Serums, einer Creme, einer Salbe oder eines Gels ist, enthaltend Polydatin als Wirkstoff.

10. Topische Zubereitung zur Verwendung nach Anspruch 9, wobei die Zubereitung in der Form einer Creme ist, enthaltend 1 bis 2 Gewichtsprozent Polydatin, vorzugsweise 1,5% Polydatin.

11. Topische Zubereitung zur Verwendung nach einem der vorstehenden Ansprüche, wobei die Zubereitung mindestens einmal täglich auf das Gesicht und den gesamten Körper aufgetragen wird, enthaltend den Bereich unter den Nägeln.

12. Topische Zubereitung zur Verwendung nach Anspruch 11, wobei die Zubereitung beginnend mindestens 24 Stunden vor dem ersten Behandlungstag mit dem Tyrosinkinaseinhibitor-Antikrebsarzneimittel aufgetragen wird.

13. Topische Zubereitung zur Verwendung nach Anspruch 12, wobei die Zubereitung mindestens einmal täglich auf das Gesicht und den gesamten Körper aufgetragen wird, beinhaltend den Bereich unter den Nägeln, beginnend eine Woche vor dem ersten Behandlungstag mit dem Tyrosinkinaseinhibitor-Antikrebsarzneimittel.

14. Topische Zubereitung zur Verwendung nach einem der Ansprüche 11-13, wobei die Zubereitung zweimal täglich aufgetragen wird.

## Revendications

1. Préparation topique comprenant le resvératrol-3-β-mono-D-glucoside (polydatine ou piceid, ou 3,5,4'-trihydroxystilbène-3-O-β-D-glucopiranoside), dans un vecteur adapté à une application sur la peau ou sur les muqueuses buccales, destinée à être utilisée dans la prévention et/ou l'atténuation d'effets indésirables cutanés ou muqueux dans le traitement par inhibiteurs de la tyrosine kinase en tant que médicaments anticancéreux, dans laquelle ladite préparation est appliquée sur la peau ou sur les muqueuses buccales une ou plusieurs fois par jour, avant et/ou pendant le traitement par lesdits médicaments.

2. Préparation topique destinée à être utilisée selon la revendication 1, dans laquelle ladite préparation est appliquée sur la peau ou sur les muqueuses buccales une ou plusieurs fois par jour en commençant au moins 24 heures avant le premier traitement par ledit médicament anticancéreux dénommé inhibiteur de la tyrosine kinase.

3. Préparation topique destinée à être utilisée selon les revendications 1 ou 2, dans laquelle lesdits effets indésirables sont choisis parmi le groupe consistant en : éruption cutanée, dermatite acnéiforme, démangeaisons, altérations unguéales, xérose cutanée, érythrodysesthésie palmoplantaire, folliculite, stomatite ou alopécie.

4. Préparation topique destinée à être utilisée selon l'une quelconque des revendications 1-3, dans laquelle lesdits médicaments anticancéreux dénommés inhibiteurs de la tyrosine kinase sont des inhibiteurs de la tyrosine kinase des récepteurs de l'EGF (EGFR-TKI).

5. Préparation topique destinée à être utilisée selon la revendication 4, dans laquelle lesdits inhibiteurs de la tyrosine kinase des récepteurs de l'EGF sont choisis parmi le groupe consistant en : erlotinib, géfitinib, afatinib et lapatinib, et les anticorps monoclonaux cétuximab et panitumumab.

6. Préparation topique destinée à être utilisée selon la revendication 5, dans laquelle ledit inhibiteur de la tyrosine kinase des récepteurs de l'EGF est l'afatinib.

7. Préparation topique destinée à être utilisée selon l'une quelconque des revendications précédentes, dans laquelle la préparation topique comprend de 0,1 à 5 % en poids de polydatine.

8. Préparation topique destinée à être utilisée selon la revendication 7, dans laquelle ladite préparation topique comprend de 1 à 2 % en poids de polydatine.

9. Préparation topique destinée à être utilisée selon l'une quelconque des revendications précédentes, dans laquelle ladite préparation se présente sous la forme d'une solution, d'un lait, d'un spray, d'une lotion, d'un sérum, d'une crème, d'un onguent ou d'un gel, contenant de la polydatine en tant que principe actif.

10. Préparation topique destinée à être utilisée selon la revendication 9, dans laquelle ladite préparation se présente sous la forme d'une crème contenant de 1 % à 2 % en poids de polydatine, de préférence de 1,5 % de polydatine.

11. Préparation topique destinée à être utilisée selon l'une quelconque des revendications précédentes, dans laquelle la préparation est appliquée au moins une fois par jour sur le visage et le corps entier, y compris sur la zone péri-unguéale.

12. Préparation topique destinée à être utilisée selon la revendication 11, dans laquelle ladite préparation est appliquée en commençant au moins 24 heures avant le premier jour de traitement par ledit médicament anticancéreux dénommé inhibiteur de la tyrosine kinase.

13. Préparation topique destinée à être utilisée selon la revendication 12, dans laquelle ladite préparation est appliquée au moins une fois par jour sur le visage et le corps entier, y compris sur la zone péri-unguéale, en commençant une semaine avant le premier jour de traitement par ledit médicament anticancéreux dénommé inhibiteur de la tyrosine kinase.

14. Préparation topique destinée à être utilisée selon l'une quelconque des revendications 11-13 dans laquelle ladite préparation est appliquée deux fois par jour.
